# FASCICULE DE BREVET EUROPEEN

(11) **EP 4 441 243 B1**
(45) Date de publication et mention de la délivrance du brevet: **08.04.2026**
(21) Numéro de dépôt: 22840252.5
(22) Date de dépôt: 30.11.2022
(51) Int. Cl.: C12Q 1/6825, C12Q 1/6806, C12Q 1/6834

(54) **FIXATION D'ACIDE NUCLÉIQUE SUR UNE ÉLECTRODE DE PLATINE**
FIXIERUNG VON NUKLEINSÄUREN AN EINE PLATINELEKTRODE
ATTACHING NUCLEIC ACIDS TO A PLATINUM ELECTRODE

(30) Priorité: 30.11.2021 FR 2112764
(43) Date de publication de la demande: 09.10.2024
(73) Titulaire: Université Paris-Saclay, 91190 Gif-sur-Yvette (FR); Centre National de la Recherche Scientifique (CNRS), 75016 Paris (FR)
(72) Inventeur: GAMBY, Jean, 91400 SACLAY (FR); LE GALL, Jeremy, 75013 PARIS (FR); KECHKECHE, Djamila, 93600 AULNAY-SOUS-BOIS (FR); POUJOULY, Claire, 91190 GIF-SUR-YVETTE (FR)
(74) Mandataire: Hautier IP
(86) Numéro de dépôt international: PCT/FR2022/052201
(87) Numéro de publication internationale: WO 2023/099841

(56) Documents cités:
- US-A1- 2020 263 241
- MD. RAHMAN ET AL: "Electrochemical DNA Hybridization Sensors Based on Conducting Polymers", SENSORS, vol. 15, no. 2, 5 February 2015 (2015-02-05), pages 3801 - 3829, XP055767739, DOI: 10.3390/s150203801
- HORNY M.-C. ET AL: "Electrochemical DNA biosensors based on long-range electron transfer: investigating the efficiency of a fluidic channel microelectrode compared to an ultramicroelectrode in a two-electrode setup", LAB ON A CHIP, vol. 16, no. 22, 1 January 2016 (2016-01-01), UK, pages 4373 - 4381, XP055933109, ISSN: 1473-0197, DOI: 10.1039/C6LC00869K
- KLEIN K ET AL: "Surface characterization and functionalization of carbon nanofibers", JOURNAL OF APPLIED PHYSICS, AMERICAN INSTITUTE OF PHYSICS, 2 HUNTINGTON QUADRANGLE, MELVILLE, NY 11747, vol. 103, no. 6, 17 March 2008 (2008-03-17), pages 61301 - 61301, XP012109526, ISSN: 0021-8979, DOI: 10.1063/1.2840049
- DAVID J.R. CONROY ET AL: "Biosensing for the Environment and Defence: Aqueous Uranyl Detection Using Bacterial Surface Layer Proteins", SENSORS, vol. 10, no. 5, 10 May 2010 (2010-05-10), pages 4739 - 4755, XP055357276, DOI: 10.3390/s100504739
- YOUNG SUN ET AL: "DNA-based Artificial Nanostructures: Fabrication, Properties, and Applications Table of Content", HANDBOOK OF NANOSTRUCTURED BIOMATERIALS AND THEIR APPLICATIONS IN NANOBIOTECHNOLOGY - VOL.2, 1 January 2005 (2005-01-01), XP055156857, Retrieved from the Internet <URL:http://cds.cern.ch/record/827525/files/0503114.pdf?version=1> [retrieved on 20141204]

## Description

### DOMAINE DE L'INVENTION

L'invention concerne la fixation d'acide nucléique sur une électrode de platine, et en particulier la fixation d'acide nucléique sur une microélectrode de platine en solution aqueuse.

L'invention concerne également une électrode de platine ainsi fonctionnalisée et l'utilisation d'une telle électrode pour la détection d'un acide nucléique cible.

### ETAT DE LA TECHNIQUE

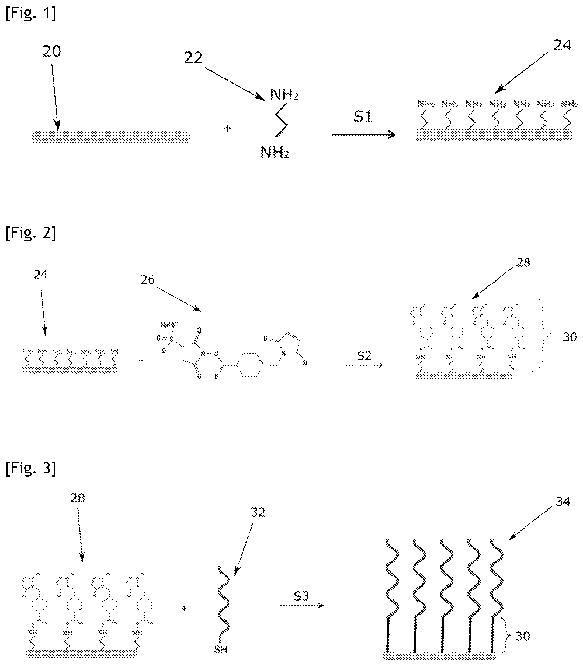

La détection de gènes par des techniques électrochimiques est connue. Ces techniques utilisent généralement une électrode en or sur laquelle est fixée une monocouche auto-assemblée (également connue sous l'expression anglaise *self-assembled monolayer* ou SAM). La fixation de cette monocouche utilise classiquement une fonction thiol car celle-ci comprend un atome de soufre qui présente une forte affinité avec l'or de l'électrode. Il est connu que la stabilité de telles monocouches auto-assemblées n'est pas satisfaisante en raison des processus de désorption chimique. Ceci est dû principalement à la nature faible de la liaison or-soufre « Au-S » pour laquelle l'énergie de liaison ~ 40 kcal/mol est très faible par rapport à une liaison plus forte de type covalente carbone-carbone « C-C » autour de 85 kcal/mol. En outre, la liaison Au-S peut être affectée par sa sensibilité à l'oxydation (changement de degré d'oxydation) ou lorsque l'électrode en or est polarisée. Par ailleurs, le processus d'oxydation de l'or est assez rapide dans des solutions aqueuses, ce qui a tendance à réduire la stabilité d'une telle électrode utilisée en milieu aqueux. MD. RAHMAN ET AL. (SENSORS, vol. 15, no. 2, (2015-02-05)) révèle un procédé de fixation sur une électrode de platine d'une sonde d'acide nucléique (AN) ayant une modification thiol. L'électrode est couverte d'une couche de polymères conducteurs (CP) (voir Fig. 1 ).

HORNY M.-C. ET AL. (LAB ON A CHIP, vol. 16, no. 22, (2016-01-01 )) décrit un système microfluidique comprenant une microélectrode et une contre électrode toutes deux avec une surface en or. Sur la première (électrode de travail) sont immobilisées des sondes d'acide nucléique ayant une modification thiol. Les deux électrodes sont placées dans un canal microfluidique en présence d'un système de mesure du courant électrique. Le système est utilisé pour la détection d'un acide nucléique cible complémentaire de la sonde. Le système est connecté pour recevoir et faire circuler des solutions (réactifs et échantillons). Le document révèle également un procédé utilisant ce système microfluidique pour la détection électrochimique d'un acide nucléique cible. Le procédé comprend en outre la mise sous tension électrique de l'électrode de travail et de la contre-électrode de sorte à maintenir une tension de travail entre elles, et la détermination d'une intensité mesurée d'un courant électrique entre l'électrode de travail et la contre-électrode. Ces documents n'apportent aucune solution pour améliorer la stabilité de l'électrode.

Il existe donc un besoin d'un dispositif de détection électrochimique présentant une meilleure stabilité de l'électrode.

### EXPOSE DE L'INVENTION

Un but de l'invention comme définie par les revendications annexées est de proposer un dispositif de détection électrochimique présentant une meilleure stabilité de l'électrode par rapport à l'art antérieur. Le but est atteint dans le cadre de la présente invention grâce à un procédé de fixation d'acide nucléique sur une électrode de platine comprenant les étapes suivantes :
- une étape (S1) de fixation d'une molécule d'éthylènediamine sur l'électrode, l'étape (S1) comprenant une électro-oxydation d'une amine primaire de la molécule d'éthylènediamine par voltammétrie cyclique,
- une étape (S2) de fixation d'une molécule sulfo SMMC de 4-(N-Maleimidomethyl)cyclohexane-1-carboxylic acid 3- sulfo-N-hydroxysuccinimide ester sur la molécule d'éthylènediamine,
- une étape (S3) de fixation d'un acide nucléique sur la molécule sulfo SMMC, l'acide nucléique étant préalablement modifié pour comprendre une fonction thiol, l'électrode étant en contact avec une solution aqueuse, c'est-à-dire avec une solution dont le solvant est l'eau, durant les étapes (S1), (S2) et (S3).

Ce procédé permet de fonctionnaliser une électrode de platine, c'est à dire dans une électrode dans un matériau qui présente une plus grande stabilité électrochimique que l'or. Un dispositif de détection électrochimique qui se fonde sur une telle électrode résout le problème mentionné plus haut.

Un tel procédé est avantageusement et optionnellement complété par les différentes caractéristiques suivantes prises seules ou en combinaison :
- la solution aqueuse est une solution physiologique ;
- l'électrode est une micro-électrode placée au moins en partie dans un canal micro-fluidique ;
- après l'étape (S3) une étape (S4) de stabilisation pendant laquelle l'électrode est placée en solution physiologique durant une durée comprise entre 20 minutes et 40 minutes, la solution présentant une concentration en NaCl comprise entre 0,4 molaire et 0,6 molaire.

L'invention porte également sur une électrode de platine pour la détection d'un acide nucléique cible comprenant une molécule d'éthylènediamine fixée sur l'électrode, une molécule sulfo SMMC de 4-(N-Maleimidomethyl)cyclohexane-1-carboxylic acid 3- sulfo-N-hydroxysuccinimide ester étant fixée sur la molécule d'éthylènediamine par électro-oxydation d'un amine primaire de la molécule d'éthylènediamine par voltammétrie cyclique, un acide nucléique sonde étant fixé sur la molécule sulfo SMMC, l'acide nucléique sonde comprenant une fonction thiol, l'acide nucléique sonde étant complémentaire de l'acide nucléique cible.

L'invention porte en outre sur un dispositif de détection d'un acide nucléique cible comprenant une électrode de platine telle que mentionnée plus haut, une contre-électrode et un système de mesures électriques relié électriquement à l'électrode de platine et à la contre-électrode.

Un tel dispositif est avantageusement et optionnellement complété par un canal micro-fluidique, l'électrode de platine étant une micro-électrode, l'électrode de platine et la contre-électrode étant placées au moins en partie dans le canal micro-fluidique.

L'invention porte aussi sur un système de détection d'acides nucléiques cibles comprenant une pluralité de dispositifs tels qu'on les a présentés plus haut, le système comprenant une entrée configurée pour recevoir une solution à analyser, l'entrée étant reliée à chaque électrode de platine de chaque dispositif.

Un tel système peut être avantageusement et optionnellement tel que deux dispositifs de la pluralité de dispositifs sont configurés pour détecter deux acides nucléiques cibles différents.

Enfin, l'invention porte sur un procédé de détection d'un acide nucléique cible dans une solution à analyser, le procédé comprenant les étapes suivantes :
- une étape (E1) de fourniture d'une électrode de travail, l'électrode de travail étant une électrode en platine comme présentée plus haut,
- une étape (E2) de fourniture d'une contre-électrode,
- une étape (E3) de mise sous tension électrique de l'électrode de travail et de la contre-électrode de sorte à maintenir une tension de travail entre elles,
- une étape (E4) de mise en contact de l'électrode de travail et de la contre-électrode avec la solution à analyser,
- une étape (E5) de détermination d'une intensité mesurée d'un courant électrique entre l'électrode de travail et la contre-électrode,
- une étape (E6) de détermination d'une présence d'un acide nucléique cible dans la solution à analyser à partir de l'intensité mesurée.

Ce procédé peut être avantageusement et optionnellement par :
- une étape de calibration comprenant les sous-étapes suivantes :
   - une première sous-étape (SE1) de fourniture de deux solutions de référence présentant des concentrations différentes en acide nucléique cible,
   - une deuxième sous-étape (SE2) de mesure pour chaque solution de référence d'une intensité de référence entre l'électrode de travail et la contre-électrode, les électrodes étant mises en contact avec la solution de référence, les électrodes étant mises sous tension électrique de sorte à maintenir la tension de travail entre elles,
   - une troisième sous étape (SE3) de détermination d'une correspondance d'intensités de courant entre l'électrode de travail et la contre-électrode en fonction et de concentrations en acide nucléique cible dans une solution à analyser,
le procédé comprenant une étape de détermination d'une concentration en acide nucléique cible dans la solution à analyser à partir de l'intensité mesurée utilisant la correspondance ;
- l'acide nucléique cible est un fragment d'acide nucléique d'un agent pathogène.

### DESCRIPTION DES FIGURES

D'autres caractéristiques et avantages de l'invention ressortiront encore de la description qui suit, laquelle est purement illustrative et non limitative, et doit être lue en regard des dessins annexés sur lesquels :
[Fig. 1]
[Fig. 2]
[Fig. 3]
   les figures 1 à 3 sont des représentation schématiques d'étapes d'un procédé de fixation d'un acide nucléique sur une électrode selon un mode de réalisation de l'invention ;
[Fig. 4] la figure 4 est une représentation schématique d'une réaction d'hybridation d'un acide nucléique cible ;
[Fig. 5] la figure 5 est une représentation schématique de mesures électrochimiques pour la détection d'un acide nucléique cible ;
[Fig. 6] la figure 6 est une représentation schématique d'un appareil de détection d'un acide nucléique ;
[Fig. 7] la figure 7 est un détail de l'appareil représenté en figure 6 ;
[Fig. 8]
[Fig. 9]

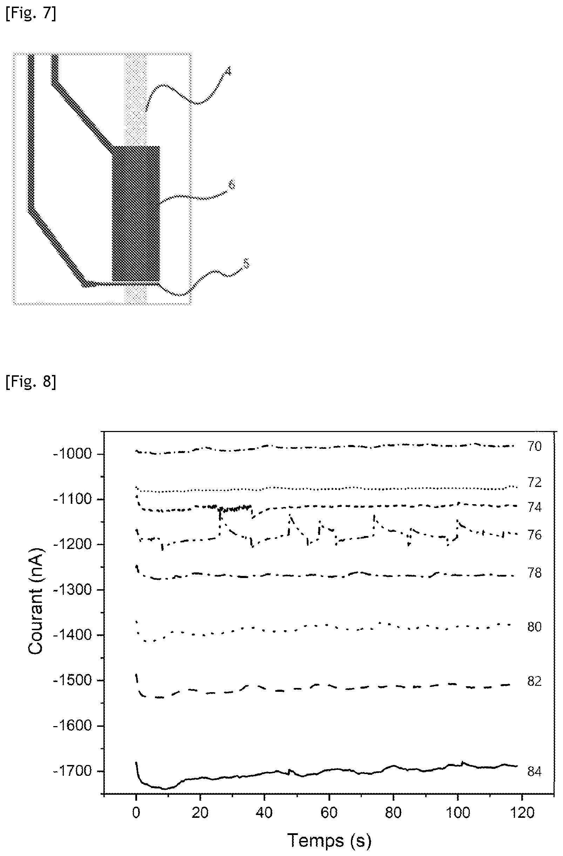

   les figures 8 à 9 sont des représentations schématiques de mesures électrochimiques pour la détection d'un acide nucléique cible;

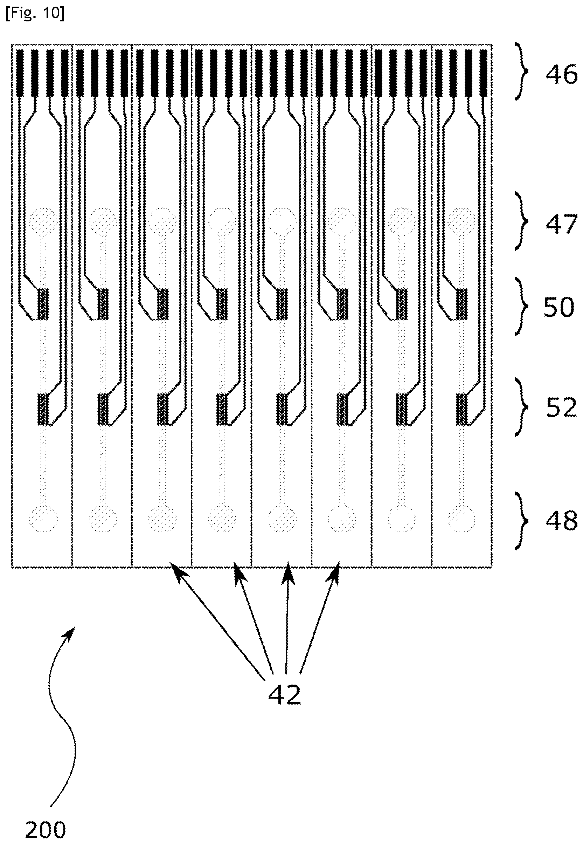

[Fig. 10] la figure 10 est une représentation schématique d'un système de détection d'acides nucléiques cibles selon un mode de réalisation de l'invention.

### DESCRIPTION DETAILLEE DE L'INVENTION

### Procédé de fixation d'acide nucléique sur une électrode de platine

En rapport avec les figures 1, 2 et 3 il est présenté un procédé selon un mode de réalisation de l'invention pour fixer un acide nucléique sur une électrode en platine.

Une électrode 20 en platine est initialement fournie. Elle est désignée également « électrode de travail » dans le texte de la description. Dans une étape préliminaire optionnelle, cette électrode peut être caractérisée pour évaluer son état de surface et ses propriétés électrochimiques.

A cette fin, deux mesures peuvent notamment être mises en œuvre : une mesure de voltammétrie cyclique et une mesure de chronoampérométrie.

Pour ces deux mesures, l'électrode 20 en platine est placée proche d'une contre-électrode, et un liquide comprenant des ions Fer(III), comme du ferricyanure de potassium, est mis en contact avec l'électrode 20 en platine et avec la contre-électrode. Par exemple, une solution de ferricyanure de potassium dans une solution aqueuse peut être utilisée La solution comprenant du ferricyanure de potassium comprend également des ions ferrocyanure Fe(II), le mélange Ferricyanure/ Ferrocyanure peut être équimolaire (1/1) ou non. La concentration en ferricyanure de potassium est choisie supérieure ou égale à 3 millimoles/litre et inférieure ou égale à 30 millimoles/litre, par exemple égale à 20 millimoles/litre. La solution comprenant des ions Fer(III) peut s'écouler sur les électrodes sous un flux choisi supérieure ou égale à 0,1 µL/seconde et inférieure ou égale à 1 µL/seconde, de préférence égal à 0,5 µL/seconde.

Une solution aqueuse est définie comme une solution dont le solvant est l'eau, c'est-à-dire dont le composant très majoritaire est l'eau. Dans cette solution on dissout généralement un sel dont les ions assurent la conductivité ionique. Cet ensemble (solution + sel) est généralement appelé électrolyte support.

Une tension électrique est appliquée entre l'électrode de travail 20 en platine et la contre-électrode et un courant électrique est mesuré entre elles.

La voltammétrie cyclique consiste à balayer la tension depuis -200 millivolts à +200 millivolts avec une vitesse de balayage de 10 millivolts/seconde et à mesurer le courant au cours du balayage.

La chronoampérométrie consiste à maintenir constante la tension, par exemple à -200 mVolts, et à mesurer le courant électrique entre les électrodes au cours d'une durée de surveillance, d'une durée par exemple de 2 minutes.

Il est à noter qu'une mesure de spectroscopie d'impédance électrochimique (EIS) peut être menée en mode potentiostatique (également connue sous la dénomination anglaise « *potentiostatic electrochemical impedance spectroscopy* » abrégée en PEIS) ou en mode galvanostatique (également connue sous la dénomination anglaise « *Galvanostatic electrochemical impedance spectroscopy* » abrégée en GEIS). Techniquement, la mesure PEIS est mise en œuvre en balayant les fréquences de 1,0 MHz à 100mHz, pour évaluer les propriétés électrochimiques de l'électrode. Sur ce, une différence de potentiel nulle est appliquée en continue entre l'électrode de platine et la contre-électrode, ainsi qu'une tension alternative de 10 millivolts, tension alternative qui est balayée en fréquence de 1,0 MHz à 100mHz.

Dans une étape S1, représentée en figure 1, une molécule d'éthylènediamine (terme abrégé ci-après en EDA) 22 est fixée sur l'électrode 20. Une solution aqueuse présentant une concentration en EDA est mise au contact de l'électrode 20 en platine. En particulier la solution aqueuse peut être une solution physiologique.

Une solution physiologique est définie comme une solution aqueuse présentant une concentration en chlorure de sodium supérieure ou égale à 0,4 mole/litre et inférieure ou égale à 0,6 mole/litre, par exemple une concentration de 0,5 mole/litre.

La concentration en EDA peut être supérieure ou égale à une millimole/litre et inférieure ou égale à 10 millimoles/litre, par exemple une concentration de 2 millimoles/litre.

Au cours de la fixation, une amine primaire NH2 de la molécule EDA réagit avec le platine, de sorte à former un produit Platine+EDA 24.

La réaction de fixation comprend une électro-oxydation d'une amine primaire de la molécule EDA, électro-oxydation qui est contrôlée par voltammétrie cyclique. La tension peut être balayée selon plusieurs cycles, par exemple dix cycles, depuis 0 Volts à +1200 mVolts.

Une électro-oxydation consiste à venir greffer de manière covalente une couche de molécules organiques grâce à une réaction électrochimique d'oxydation ou de réduction d'un groupe chimique particulier sur la surface d'une électrode. Ici une électro-oxydation de l'amine primaire sur l'électrode de platine permet de réaliser un électrogreffage de l'éthylènediamine. Une liaison covalente est ainsi créée entre un azote de l'éthylènediamine et la surface de l'électro de platine. Plusieurs molécules EDA peuvent être fixées sur l'électrode 20 au cours de l'étape S1, de sorte qu'une monocouche auto-assemblée de molécules EDA est formée à la surface de l'électrode.

A l'issue de l'étape S1, une étape de caractérisation de l'électrode Platine+EDA 24 peut être mise en œuvre, en utilisant l'une des mesures précédemment décrites : voltammétrie cyclique et/ou chronoampérométrie.

Dans une étape S2, représentée en figure 2, une molécule sulfo SMMC de 4-(N-Maleimidomethyl)cyclohexane-1-carboxylic acid 3- sulfo-N-hydroxysuccinimide ester 26 est fixée sur la molécule d'éthylènediamine 22 précédemment fixée sur l'électrode 20 de platine.

Plus précisément, la molécule sulfo SMMC 26 est fixée à une extrémité distale de la molécule EDA 22, cette extrémité distale étant opposée à une extrémité proximale de la molécule EDA qui est, elle, fixée à l'électrode de platine 20. La molécule sulfo SMMC 26 présente une fonction N-hydroxysuccinimide (communément abrégée en « NHS ») qui réagit avec une deuxième amine de la molécule EDA, de sorte à former un produit Platine+EDA+sulfo SMMC 28. La deuxième amine de la molécule EDA constitue l'extrémité distale.

Pour cette étape S2, une solution aqueuse présentant une concentration en molécule sulfo SMMC est mise au contact de l'électrode Platine+EDA 24. En particulier la solution aqueuse peut être une solution physiologique.

La concentration en molécule sulfo SMMC peut être supérieure ou égale à une millimole/litre et inférieure ou égale à 100 millimoles/litre, par exemple une concentration de dix millimoles/litre.

Cette réaction de fixation peut notamment être réalisée à pH supérieur ou égal à 7,5 et inférieur ou égal à 9 et en statique sur une durée supérieure ou égale à 45 minutes et inférieure ou égale à 1h15, de préférence égale à 1 heure. Une réaction en statique signifie ici qu'une quantité de solution comprenant des molécules sulfo SMCC, quantité monobloc et fixe, est mise en contact avec l'électrode en platine. En particulier, il n'y a pas de flux de solution qui s'écoule sur l'électrode de platine.

Au cours de l'étape S2, plusieurs molécules sulfo SMCC peuvent être fixées chacune sur une molécule EDA fixée à l'électrode 20. La monocouche auto-assemblée de molécules EDA précédemment évoquée est alors complétée des molécules sulfo SMCC.

L'ensemble EDA+sulfo SMCC fixé au-dessus de l'électrode est désigné sous le terme de linker (c'est-à-dire « lien » en anglais) référencé 30 sur la figure 2.

A l'issue de l'étape S2, une étape de caractérisation de l'électrode Platine+EDA+sulfo SMCC 28 peut être mise en œuvre, en utilisant l'une des mesures précédemment décrites : voltammétrie cyclique et/ou chronoampérométrie et/ou PEIS.

Dans une étape S3, représentée en figure 3, un acide nucléique 32 est fixée sur la molécule sulfo SMMC 26 précédemment fixée sur l'électrode 20 de platine via la molécule EDA 22.

Plus précisément, l'acide nucléique 32 est fixé à une extrémité distale de la molécule sulfo SMMC 26 opposée à une extrémité proximale de la molécule sulfo SMMC 26 qui est fixée à l'extrémité distale de la molécule EDA 22.

L'acide nucléique 32 est préalablement modifié pour comprendre à l'une de ses extrémités une fonction thiol, c'est-à-dire une fonction -SH comprenant un atome de soufre S et un atome d'hydrogène H. C'est cette fonction thiol qui permet de fixer l'acide nucléique à la molécule sulfo SMMC 26, et plus précisément sur une fonction maléimide de la molécule sulfo SMMC 26.

Pour cette étape S3, une solution aqueuse présentant une concentration en acide nucléique est mise au contact de l'électrode Platine+EDA+sulfo SMCC 28. En particulier la solution aqueuse peut être une solution physiologique.

La concentration en molécule acide nucléique peut être supérieure ou égale à 0.1 micromole/litre et inférieure ou égale à 10 micromoles/litre, par exemple une concentration de 1 micromole/litre.

Cette réaction de fixation de l'étape S3 peut notamment être réalisée en statique sur une durée supérieure ou égale à 1h45 minutes et inférieure ou égale à 2h15, de préférence égale à 2 heures.

Une fois l'acide nucléique 32 fixé sur le « linker » 30, il est formé un produit Platine+EDA+sulfo SMMC+acide nucléique 34.

La réaction de fixation de l'étape S3 peut notamment être réalisée en statique, c'est-à-dire sans flux d'une solution d'acide nucléique, ou en quasi-statique c'est-à-dire avec un flux quasiment nul, par exemple un flux supérieur ou égal à 0,01 µL/s et inférieure ou égal à 0,05 µL/s.

Au cours de l'étape S3, plusieurs acides nucléiques peuvent être fixés chacun sur une molécule sulfo SMMC 26 fixée sur l'électrode 20 de platine via la molécule EDA 22. La monocouche auto-assemblée de molécules EDA complétée des molécules sulfo SMCC précédemment évoquée est alors complétée des acides nucléiques.

Une étape S4 de stabilisation peut être optionnellement mise en œuvre après l'étape S3. Durant cette étape S4, l'électrode est placée en solution physiologique durant une durée comprise entre 20 minutes et 40 minutes, idéalement 30 minutes.

A l'issue de l'étape S3 et le cas échéant de l'étape S4, une étape de caractérisation de l'électrode Platine+EDA+sulfo SMMC+acide nucléique 34 peut être mise en œuvre, en utilisant l'une des mesures précédemment décrites : voltammétrie cyclique et/ou chronoampérométrie et/ou PEIS.

Le procédé de fixation d'un acide nucléique sur une électrode de platine tel qu'on l'a présenté utilise la fixation du « linker » sur l'électrode de platine qui permet ensuite de fixer l'acide nucléique via une fonction thiol.

L'utilisation de la fonction thiol pour fixer directement l'acide nucléique sur l'électrode en platine n'est pas satisfaisante, l'affinité étant faible entre le platine et cette fonction.

L'ensemble des étapes du procédé décrit ici s'effectue en solution aqueuse, ce qui permet de travailler avec des acides nucléiques et de les fixer sur l'électrode. Il n'est en particulier pas utilisé de solvant organique, un tel solvant pouvant dégrader l'acide nucléique et empêcher sa fixation sur l'électrode de platine.

### Electrode de platine pour la détection d'un acide nucléique cible

Pour la détection d'un acide nucléique cible, il est possible d'utiliser une électrode en platine sur laquelle est fixée un brin d'acide nucléique sonde complémentaire de l'acide nucléique cible.

A cet effet, l'objet de l'invention est aussi une électrode de platine qui comprend une molécule d'éthylènediamine 22 fixée sur l'électrode, une molécule sulfo SMMC 26 de 4-(N-Maleimidomethyl)cyclohexane-1-carboxylic acid 3- sulfo-N-hydroxysuccinimide ester fixée sur la molécule d'éthylènediamine 22, un acide nucléique sonde 32 étant fixé sur la molécule sulfo SMMC 26, l'acide nucléique sonde 32 comprenant une fonction thiol, l'acide nucléique sonde 32 étant complémentaire de l'acide nucléique cible 36.

Une telle électrode peut être produite par le procédé de fixation d'acide nucléique présenté précédemment dans le cas où l'acide nucléique fixé est l'acide nucléique sonde complémentaire de l'acide nucléique cible 36.

L'électrode de platine peut être notamment une micro-électrode.

Une micro-électrode est définie ici comme une électrode dont au moins des dimensions est inférieure ou égale à 1 000 µm.

Le procédé de fixation décrit précédemment peut être mis en œuvre pour différents types d'électrode de platine, et en particulier pour une micro-électrode.

La micro-électrode peut être produite par photolithographie.

En particulier du platine peut être déposé sur un substrat en verre sous forme d'une piste obtenue après des étapes de dépôt et lithographie en salle blanche. L'électrode est comprise sur cette piste de platine.

La micro-électrode présente une longueur dans une première direction et une largeur dans une deuxième direction perpendiculaire à la première direction. Les dimensions de la micro-électrode de travail peuvent être par exemple de 30 µm en largeur et 300 µm en longueur.

Pour les étapes de caractérisation d'électrode décrites précédemment en rapport avec le procédé de fixation, une contre-électrode peut être placée proche de l'électrode de platine. Cette contre-électrode peut être fabriquée simultanément avec l'électrode platine par dépôt sur le substrat en verre par lithographie en salle blanche.

Les dimensions de la contre-électrode sont choisies plus importantes que les dimensions de l'électrode de travail, par exemple de 2 000 µm en largeur et 300 µm en longueur. De telles dimensions font alors de la contre-électrode une micro-électrode.

Une telle micro-électrode peut être placée au moins en partie dans un canal micro-fluidique.

Un canal micro-fluidique est un canal permettant l'écoulement de fluides et présentant des dimensions dans un plan transverse à la direction d'écoulement général des fluides qui sont supérieures ou égales à 100 µm et inférieures ou égales à 500 µm.

Le canal présente dans un plan transverse à la direction d'écoulement général des fluides, une largeur dans le plan de l'électrode et une hauteur perpendiculaire au plan de l'électrode.

Il est possible de mettre en œuvre toutes les étapes du procédé de fixation d'un acide nucléique sur une micro-électrode placée au moins en partie dans un canal micro-fluidique.

De préférence, le canal est placé sur la micro-électrode de travail de sorte que la direction d'écoulement est perpendiculaire à la longueur de l'électrode. Si la longueur de l'électrode est plus importante que la largeur du canal micro-fluidique, alors la longueur effective de l'électrode c'est-à-dire la longueur vue par le liquide transporté dans le canal est fixée par la largeur du canal. Par exemple avec une largeur du canal de 300 µm, la longueur effective de l'électrode de travail est de 300 µm pour une largeur de 30 µm, la longueur effective de la contre-électrode est de 300 µm pour une largeur de 2000 µm.

Plus précisément, le fait que l'ensemble des étapes du procédé de fixation s'effectue en solution aqueuse, permet de travailler en canal micro-fluidique. Il n'est en particulier pas utilisé de solvant organique ce qui aurait empêché de travailler en canal micro-fluidique, la structure de celui-ci et ses raccords indispensables à son alimentation en fluide pouvant être détériorée par un solvant organique.

Il est à noter que travailler avec un canal micro-fluidique permet de travailler dans des régimes d'écoulement contrôlables pour notamment en contrôler la convection. Cela présente l'avantage d'obtenir plus rapidement certaines réactions (ayant lieu dans l'épaisseur de la couche de diffusion créée à la surface de l'électrode) en diminuant le temps de diffusion vers l'électrode. Les cibles d'acides nucléiques, présentes dans le liquide s'écoulant dans le canal, rentrent plus rapidement en collision avec la surface de l'électrode pour engager des réactions d'hybridation avec les acides nucléiques sondes fixés à l'électrode de platine.

Pour placer une micro-électrode au moins en partie dans un canal micro-fluidique, le canal micro-fluidique peut être fabriqué dans une résine PDMS, sous la forme par exemple d'une rainure ouverte à la surface d'une pièce de résine PDMS. La rainure s'étend selon une direction d'extension du canal micro-fluidique.

Dans un plan perpendiculaire à cette direction d'extension, la rainure peut présenter une section rectangulaire. Dans ce cas et comme la rainure est ouverte, la résine PDMS définit seulement trois côtés du rectangle de la section rectangulaire.

La résine PDMS est ensuite collée sur le substrat portant la micro-électrode, par exemple un substrat de verre, de façon à ce que le substrat ferme le canal micro-fluidique.

Dans le cas où la rainure présente une section rectangulaire, le canal micro-fluidique présente une section rectangulaire dont trois côtés sont formés par la résine PDMS et le dernier côté est formé par le substrat.

La résine PDMS est ajustée en position de sorte que l'électrode la piste se situe au moins en partie dans le canal micro-fluidique.

D'autres matériaux polymères autre que la résine PDMS peuvent être utilisés pour le canal microfluidique comme les polymères : PMMA, PMP, PVDF, COC, PET ; ou les copolymères à blocs, etc...

D'autres technologies de fabrication peuvent être utilisées pour la fabrication du micro-canal comme la gravure chimique sur verre, le moulage, l'embossage, ou l'impression 3-D.

Les différentes solutions apportant les réactifs peuvent être insérées dans le canal micro-fluidique pour réaliser les différentes étapes du procédé décrit précédemment.

Pour les étapes de caractérisation d'électrode, une contre-électrode peut être préalablement placée dans le canal micro-fluidique proche de l'électrode de platine.

### Dispositif de détection d'un acide nucléique cible

L'électrode de platine pour la détection d'un acide nucléique cible présentée plus haut peut notamment être comprise dans un dispositif de détection d'un acide nucléique cible, le dispositif comprenant en outre une contre-électrode et un système de mesures électriques relié électriquement à l'électrode de platine et à la contre-électrode.

L'électrode de platine sur laquelle est fixée l'acide nucléique sonde est nommée ci-après électrode de travail.

La contre-électrode est placée suffisamment proche de l'électrode de travail de sorte à pouvoir imposer une différence de potentiel entre ces électrodes et mesurer un courant entre elles.

Le dispositif comprend à cet effet un système de mesures électriques configuré pour imposer une différence de potentiel entre les électrodes, mesurer cette différence de potentiel et mesurer un courant entre les électrodes.

Le dispositif est configuré de sorte qu'une solution à analyser peut être mise en contact simultané avec l'électrode de travail et la contre-électrode.

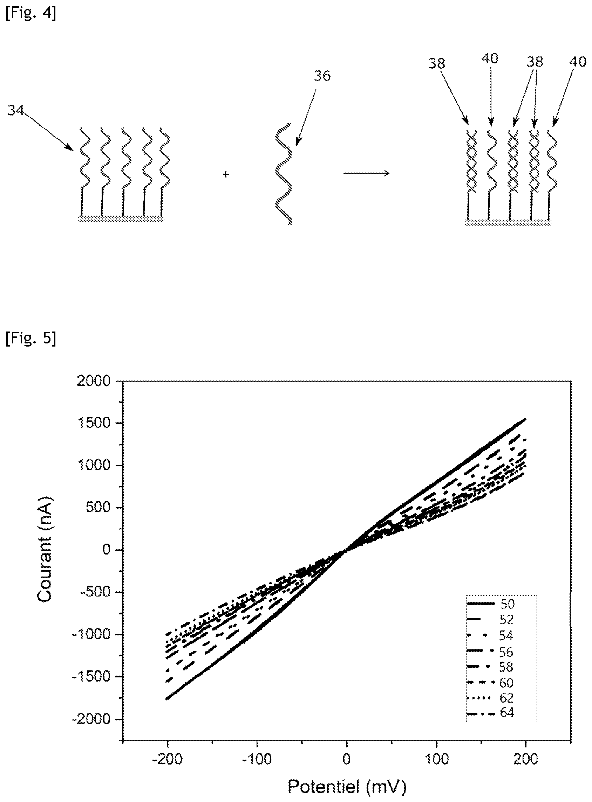

Lorsque la solution à analyser contient des acides nucléiques cibles, une réaction d'hybridation entre l'acide nucléique cible et l'acide nucléique sonde se produit sur l'électrode de travail, comme illustré en figure 4.

L'électrode de travail comprend un ensemble fonctionnalisé 34 électrode Platine+EDA+sulfo SMMC+acide nucléique comme illustré en figure 4. Cet ensemble fonctionnalisé 34 comprend un acide nucléique sonde qui est complémentaire d'un acide nucléique cible 36.

La réaction d'hybridation se produit lorsque l'ensemble fonctionnalisé 34 est mis en présence d'une solution contenant des acides nucléiques cibles 36. L'acide nucléique 36 cible et l'acide nucléique sonde 32 de l'ensemble fonctionnalisé 34 s'hybrident.

De cette manière un hybride 38 « acide nucléique sonde 32+ acide nucléique cible 36 » apparaît au-dessus de l'électrode de platine. Les propriétés électrochimiques de l'électrode sont modifiées par cette hybridation de sorte que la présence d'acide nucléique cible 36 peut être détectée.

Lorsque l'électrode de platine comprend une pluralité d'acides nucléiques sondes, la présence d'acide nucléique 36 cible produit un ou plusieurs hybrides 38 « acide nucléique sonde 32+ acide nucléique cible 36 » au-dessus de l'électrode de platine. D'autres acides nucléiques sondes qui ne subissent pas d'hybridation restent des groupements non hybridés 40. Les propriétés électrochimiques de l'électrode sont modifiées par l'hybridation en fonction de la proportion entre hybrides 38 et groupements non hybridés 40. Ces modifications permettent d'estimer la concentration en acide nucléiques cible dans la solution à analyser.

La réaction d'hybridation est préférentiellement réalisée en statique sur une durée supérieure ou égale à 15 minutes et inférieure ou égale à 1h, de préférence égale à 30 minutes.

La modification des propriétés électrochimiques de l'électrode peut être mise en évidence par le système de mesures électriques.

Les graphes illustrés en figure 5 montrent une telle modification.

Les graphes ont été obtenus expérimentalement en utilisant une électrode de platine pour la détection d'un acide nucléique cible comme il a été décrit précédemment. Plus précisément il s'agit d'une électrode présentant une monocouche auto-assemblée de molécules EDA complétée des molécules sulfo-SMCC et des acides nucléiques sondes complémentaires de l'acide nucléique cible.

L'électrode est utilisée dans un dispositif de détection comme décrit, c'est à dire comprenant en outre une contre-électrode et un système de mesures électriques relié électriquement à l'électrode de platine et à la contre-électrode.

Différentes solutions de référence présentant une concentration de l'acide nucléique cible égale respectivement à 10⁻¹⁸, 10⁻¹⁶, 10⁻¹⁴, 10⁻¹², 10⁻¹⁰,10⁻⁸ et 10⁻⁶ mole/litre ont été employées.

Une solution de référence neutre ne comprenant pas l'acide nucléique cible a également été employée.

Pour chaque solution, la mesure électrochimique suivante est mise en œuvre : on fait varier la différence de potentiel (VT-VCE) entre le potentiel VT de l'électrode de travail et le potentiel VCE de la contre-électrode et on mesure l'intensité électrique entre ces électrodes.

La différence de potentiel (VT-VCE) est reportée sur l'axe des abscisses de la figure 5, l'intensité électrique sur l'axe des ordonnées.

La différence de potentiel (VT-VCE) est balayée entre -200 millivolts et +200 millivolts.

Initialement, les électrodes sont mises au contact de la solution de référence pour effectuer la mesure électrochimique décrite plus haut et illustrée par la courbe 50.

La courbe 50 apparaît comme une courbe quasi-linéaire sur la figure 5.

Ensuite, d'autres mesures électrochimiques en utilisant les autres solutions de référence sont mises en œuvre avec le même dispositif.

Les courbes respectives 52, 54, 56, 58, 60, 62 et 64 représentent les cas où la solution de référence présente une concentration en acides nucléiques cibles égales respectivement à 10⁻¹⁸, 10⁻¹⁶, 10⁻¹⁴, 10⁻¹², 10⁻¹⁰,10⁻⁸ et 10⁻⁶ mole/litre. Après chaque mesure électrochimique, les électrodes sont rincées avec une solution physiologique c'est-à-dire une solution de référence neutre.

Une mesure avec Fe(II)/Fe(III) est réalisée pour s'assurer que la surface de l'électrode de travail a bien été modifiée d'une part par les SAM le cas échéant et d'autre part par les acides nucléiques sonde et cible.

Les électrodes sont à nouveau rincées avec une solution physiologique puis on procède à une mesure électrochimique avec une nouvelle solution de référence présentant une concentration en acide nucléique cible non nulle.

On peut veiller à effectuer les mesures dans l'ordre croissant des concentrations en acide nucléique des solutions de référence.

Il est également possible de changer le jeu des deux électrodes (électrode de travail et contre-électrode) entre chaque mesure.

Les courbes 52, 54, 56, 58, 60 et 62 apparaissent également proche de courbes linéaires sur la figure 5, et la pente de cette courbe est de plus en plus faible à mesure que la concentration en acides nucléiques cibles augmente. Autrement dit, les propriétés électrochimiques de l'électrode sont modifiées en présence d'acides nucléiques cibles, la modification étant d'autant plus importante que la concentration en acides nucléiques cibles est importante.

Un dispositif de détection d'un acide nucléique cible tel que présenté précédemment permet de détecter la présence ou l'absence d'un acide nucléique dans une solution mise au contact de ses électrodes.

Cette expérience a été menée avec des séquences ADN sondes pour détecter des cibles ARN codants (E, N, RdRp) et une séquence de contrôle négatif. La table 1 mentionne les différentes séquences utilisées.

**Table 1 : séquences utilisées pour produire une sonde d'acide nucléique sur l'électrode de travail. Les ADN Sondes sont modifiées par une fonction thiol (-SH) en position 5'.**

| **Nom de la sonde** | **Séquence ADN / ARN correspondante** |
|---|---|
| ADN Sonde - Gène E | 5' - TCG-CTA-TTA-AGT-ATT-AAC-GTA-CCT-GT - 3' |
| ADN Sonde - Gène N | 5' - GAT-TGC-GGG-TGC-CAA-TGT-G - 3' |
| ADN Sonde - Gène RdRP | 5' - CCG-CCA-CAC-ATG-ACC-ATC-TCA-C - 3' |
| ARN Cible - Gène E | 5' - ACA-GGU-ACG-UUA-AUA-CUU-AAU-AGC-GU - 3' |
| ARN Cible - Gène N | 5' - CAC-AUU-GGC-ACC-CGC-AAU-C - 3' |
| ARN Cible - Gène RdRP | 5' - GUG-AGA-UGG-UCA-UGU-GUG-GCG-G - 3' |
| ADN Cible - poly(A) | 5' - AAA-AAA-AAA-AAA-AAA-AAA-AAA-AAA-AA - 3' |

En option, le dispositif comprend en outre un canal micro-fluidique, l'électrode de platine et la contre-électrode étant des micro-électrodes, l'électrode de platine et la contre-électrode étant placées au moins en partie dans le canal micro-fluidique.

Le dispositif est configuré de sorte qu'en fonctionnement, des fluides peuvent parcourir le canal micro-fluidique et entrer en contact avec l'électrode de travail et la contre-électrode.

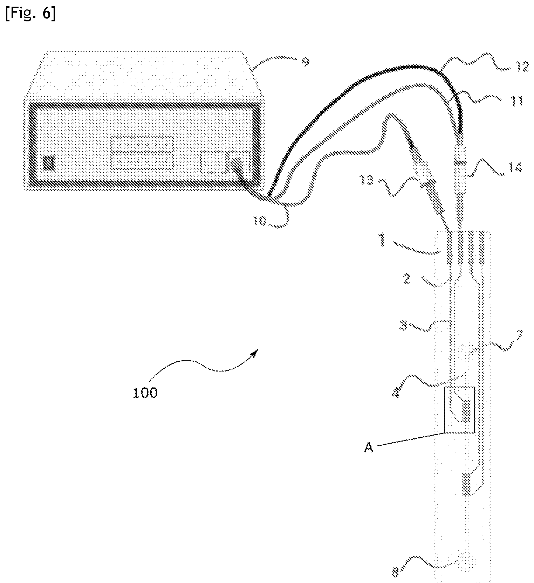

La figure 6 représente schématiquement un exemple de dispositif 100 de détection d'un acide nucléique cible.

Le dispositif 100 comprend un substrat en verre 1 sur lequel est déposé du platine sous forme de deux pistes métalliques 2 et 3, dont une piste de travail 2 et une piste de référence 3. Ce dépôt peut notamment être réalisé par des étapes de dépôt et lithographie en salle blanche comme mentionné précédemment.

La piste de travail 2 présente une terminaison métallique nommée électrode de travail 5. C'est sur cette électrode de travail 5 qu'est fixé l'acide nucléique sonde.

La piste de référence 3 présente une terminaison métallique nommée contre-électrode 6.

L'électrode de travail 5 et la contre-électrode 6 sont visibles sur la figure 7 qui est un agrandissement de la zone A de la figure 6.

Un canal micro fluidique 4 de section rectangulaire est formé par collage d'une résine PDMS sur le substrat 1 de verre. Le canal micro fluidique 4 est localisé par rapport au substrat de façon à intégrer une partie des terminaisons métalliques des pistes métalliques 2 et 3.

Les dimensions du canal micro fluidique 4, de l'électrode de travail 5 et de la contre-électrode 6 sont ajustées de sorte que seule une partie de l'électrode de travail 5 et seule une partie de la contre-électrode 6 sont comprises dans le canal micro-fluidique.

De plus la surface de la contre-électrode 6 dans le canal 4 est beaucoup plus importante que la surface de l'électrode de travail 5 dans le canal 4. Cela permet d'utiliser la contre-électrode 6 non seulement comme une contre-électrode mais aussi comme une pseudo-référence.

Le canal micro fluidique 4 présente une entrée 7 et une sortie 8. Les fluides introduits par l'entrée 7 traversent le micro-canal 4 jusqu'à la sortie 8. Au cours de cette traversée, les fluides entrent en contact avec l'électrode de travail 5 et la contre-électrode 6. Les fluides n'entrent pas en contact avec le reste des pistes métalliques 2 et 3.

Le dispositif 100 comprend un système de mesures électriques configuré pour imposer une différence de potentiel entre les électrodes, mesurer cette différence de potentiel et mesurer un courant entre les électrodes.

Le système de mesures électriques peut être notamment formé des éléments suivants:
- un instrument 9 de type potentiostat et constituant l'électromètre du potentiostat, l'instrument 9 possédant trois sorties à savoir une sortie de travail 10, une sortie de référence 11 et une sortie de contre-électrode 12,
- un connecteur de travail 13 reliant la sortie de travail 10 à la piste métallique 2,
- un connecteur de référence 14 reliant la sortie de référence 11 et la sortie de contre-électrode 12 à la piste de référence 3.

### Système de détection comprenant une pluralité de dispositifs de détection

Un autre objet de l'invention est un système de détection d'acides nucléiques cibles comprenant une pluralité de dispositifs de détection d'acides nucléiques tels qu'on vient de les présenter.

Le système comprend une entrée configurée pour recevoir une solution à analyser, l'entrée étant reliée à chaque électrode de platine de chaque dispositif. Autrement dit une solution à analyser qui est introduite dans l'entrée du système se déplace dans le système jusqu'à entrer dans chaque dispositif et entrer en contact avec l'électrode de travail et la contre-électrode.

Les dispositifs sont de préférence disposés en parallèle, c'est-à-dire que l'entrée du système est reliée directement à chaque électrode de platine de chaque dispositif. Une solution à analyser qui est introduite dans l'entrée du système se déplace dans le système jusqu'à entrer simultanément dans chaque dispositif et entrer en contact avec l'électrode de travail et la contre-électrode.

Un système de détection permet de réaliser plusieurs mesures simultanément. Par exemple, chaque détecteur peut être configuré pour détecter le même acide nucléique. L'introduction d'une solution à analyser permet de réaliser plusieurs fois la mesure de détection voire de mesure de la concentration en acides nucléiques. Un tel système de détection permet ainsi d'obtenir une mesure de meilleure précision.

La figure 10 représente schématiquement un exemple de système de détection 200, qui comprend huit dispositifs 42 pour la détection. Chaque dispositif de détection comprend une entrée de liquide 47 et une sortie de fluide 48. Les entrées 47 peuvent être toutes reliées chacune directement à l'entrée du système pour obtenir une configuration en parallèle. Chaque dispositif de détection comprend ici deux ensembles 50, 52 « électrode de travail et contre-électrode ». Chaque électrode est reliée à une zone de connexion électrique 46 qui permet de connecter électriquement l'électrode à son système de mesures électriques.

Le système de détection peut avantageusement comprendre deux dispositifs de configurés pour détecter deux acides nucléiques cibles différents. Dans ce cas, le système de détection permet de réaliser simultanément des mesures concernant des acides nucléiques différents.

En particulier, chaque dispositif du système peut être configuré pour détecter un acide nucléique cible différent des acides nucléiques que les autres dispositifs sont configurés pour détecter. Dans ce cas, le système de détection permet de réaliser simultanément des mesures concernant autant d'acides nucléiques différents qu'il y a de dispositifs dans le système.

### Procédé de détection d'un acide nucléique cible

Un autre objet de l'invention est un procédé de détection d'un acide nucléique cible dans une solution à analyser comprenant les étapes suivantes :
- une étape E1 de fourniture d'une électrode de travail, l'électrode de travail étant une électrode en platine pour la détection d'un acide nucléique tel qu'on a pu le présenter précédemment,
- une étape E2 de fourniture d'une contre-électrode,
- une étape E3 de mise sous tension électrique de l'électrode de travail et de la contre-électrode de sorte à maintenir une tension de travail entre elles,
- une étape E4 de mise en contact de l'électrode de travail et de la contre-électrode avec la solution à analyser,
- une étape E5 de détermination d'une intensité mesurée d'un courant électrique entre l'électrode de travail et la contre-électrode,
- une étape E6 de détermination d'une présence d'un acide nucléique cible dans la solution à analyser à partir de l'intensité mesurée.

Les étapes E1 et E2 du procédé de détection peuvent être mis en œuvre par la fourniture d'un dispositif de détection d'un acide nucléique cible tel qu'on a pu le décrire précédemment.

L'étape E3 peut être mise en œuvre grâce à un système de mesures électriques configuré pour imposer une différence de potentiel entre les électrodes, mesurer cette différence de potentiel et mesurer un courant entre les électrodes. Typiquement, la différence de potentiel (VT-VCE) entre le potentiel VT de l'électrode de travail et le potentiel VCE de la contre-électrode peut être choisie supérieure ou égale à -250 millivolts et inférieure ou égale à +250 millivolts, de préférence supérieure ou égale à -250 millivolts et inférieure ou égale -150 millivolts, de préférence supérieure ou égale à -220 millivolts et inférieure ou égale -180 millivolts, de préférence égale à -200 millivolts.

L'étape E4 de mise en contact de l'électrode de travail et de la contre-électrode avec la solution à analyser peut être effectuée en statique ou bien en dynamique. En statique, une quantité de solution monobloc et fixe est mise en contact avec les électrodes de sorte à mettre en contact électrique les électrodes par la solution.

En dynamique, un flux continu de solution à analyser passe au contact des électrodes de sorte à mettre en contact électrique les électrodes par le flux de solution. Cette mise en œuvre dynamique peut notamment être effectuée par un dispositif de détection d'un acide nucléique dans l'option où il comprend un canal micro-fluidique. Un flux de solution à analyser peut parcourir le canal et passer au contact des électrodes. Typiquement, un flux de solution peut être généré dans un canal micro-fluidique avec une valeur choisie supérieure ou égale à 0,1 µL/seconde et inférieure ou égale à 1 µL/seconde, de préférence égal à 0,5 µL/seconde.

Dans tous les cas, la mise en contact avec la solution à analyser est adaptée pour produire un contact électrique entre les électrodes par la solution à analyser. La solution à analyser est une solution aqueuse et peut en particulier être une solution physiologique.

A l'occasion de cette étape E4 et lorsque la solution à analyser contient des acides nucléiques cibles, une réaction d'hybridation entre l'acide nucléique cible et l'acide nucléique sonde se produit sur l'électrode de travail, comme présenté précédemment en rapport avec la figure 4.

Les propriétés électrochimiques de l'électrode sont modifiées par cette hybridation de sorte que la présence d'acide nucléique cible dans la solution à analyser peut être détectée.

L'étape E5 a lieu lorsque l'électrode de travail et la contre-électrode sont mises en contact avec la solution à analyser de sorte à produire un contact électrique entre les électrodes par la solution à analyser.

L'étape E5 de détermination d'une intensité mesurée d'un courant électrique entre l'électrode de travail et la contre-électrode peut être mise en œuvre grâce au système de mesures électriques.

L'étape E5 peut notamment être mise en œuvre par une mesure de chronoampérométrie.

La figure 8 illustre un exemple de mesure de chronoampérométrie.

Les graphes illustrés en figure 8 ont été obtenus expérimentalement pour différentes solutions de référence, en fixant la différence de potentiel (VT-VCE) entre le potentiel VT de l'électrode de travail et le potentiel VCE de la contre-électrode à -200 millivolts et en mesurant l'intensité électrique entre ces électrodes au cours du temps.

L'intensité électrique entre les électrodes est reportée sur l'axe des ordonnées, l'axe des abscisses correspondant au temps.

La courbe 84 représente le cas où la solution à analyser est une solution de référence qui ne comprend pas d'acides nucléiques.

La mesure de la courbe 84 est réalisée avant les autres.

Les courbes respectives 82, 80, 78, 76, 74, 72 et 70 représentent les cas où la solution de référence présente une concentration en acides nucléiques cibles égales respectivement à 10⁻¹⁸, 10⁻¹⁶, 10⁻¹⁴, 10⁻¹², 10⁻¹¹,10⁻⁸ et 10⁻⁶ mole/litre. Après chaque mesure électrochimique, les électrodes sont rincées avec une solution physiologique c'est-à-dire une solution de référence neutre. On peut veiller à effectuer les mesures dans l'ordre croissant des concentrations en acide nucléique des solutions de référence.

La figure 8 montre que la valeur moyenne au cours du temps de l'intensité mesurée permet de différencier les différentes concentrations en acides nucléiques cibles : plus la valeur moyenne est élevée, et plus la concentration est importante.

L'étape E6 de détermination de la présence en acide nucléique cible dans la solution à analyser peut être effectuée en comparant l'intensité mesurée à une intensité de référence ou intensité sonde mesurée préalablement pour une solution de référence ne contenant aucun acide nucléique cible. Si l'intensité mesurée est significativement différente de l'intensité sonde, alors l'acide nucléique cible est présent dans la solution à analyser. Une différence significative est une différence qui passe un certain seuil. Ce seuil peut être évalué à partir d'une statistique de mesures de ce type permettant d'en évaluer l'écart-type et le bruit de fond. Le seuil peut être par exemple choisi égal à un écart-type ou plusieurs écart-types par exemple trois écart-types.

En option, le procédé comprend en outre une étape de calibration comprenant les sous-étapes suivantes :
- une première sous-étape SE1 de fourniture de deux solutions de référence présentant des concentrations différentes en acide nucléique cible,
- une deuxième sous-étape SE2 de mesure pour chaque solution de référence d'une intensité de référence entre l'électrode de travail et la contre-électrode, les électrodes étant mises en contact avec la solution de référence, les électrodes étant mises sous tension électrique de sorte à maintenir la tension de travail entre elles,
- une troisième sous étape SE3 de détermination d'une correspondance d'intensités de courant entre l'électrode de travail et la contre-électrode en fonction et de concentrations en acide nucléique cible dans une solution à analyser,
le procédé comprenant une étape de détermination d'une concentration en acide nucléique cible dans la solution à analyser à partir de l'intensité mesurée en utilisant la correspondance.

Au cours de la première sous-étape SE1 deux solutions de référence présentant des concentrations différentes et connues en acide nucléique cible sont fournies.

Préférentiellement un nombre de solutions de référence supérieur à deux présentant sont fournies : plus ce nombre est important et meilleure est la calibration.

Par exemple, les solutions de référence peuvent présenter les concentrations en acide nucléique cible égales à 10⁻¹⁸, 10⁻¹⁶, 10⁻¹⁴, 10⁻¹², 10⁻¹⁰,10⁻⁸ et 10⁻⁶ mole/litre.

Au cours de la deuxième sous-étape SE2 chaque solution de référence est successivement utilisée pour mettre en contact l'électrode de travail et la contre-électrode de sorte à établir en contact électrique entre elles.

Les électrodes sont mises sous tension électrique de sorte à maintenir la tension de travail entre elles, par exemple -200 millivolts.

Comme pour l'étape E5 du procédé de détection d'acide nucléique cible, une intensité entre les électrodes est mesurée. Cette intensité est dénommée intensité de référence étant donné qu'elle est déterminée pour une des solutions de référence dont la concentration en acides nucléiques est connue.

La détermination peut notamment passer par le calcul d'une intensité moyenne au cours du temps obtenue par chronoampérométrie.

La figure 8 précédemment décrite correspond à la détermination de huit intensités de référence, chacune étant associé à une concentration en acide nucléique connue.

Au cours de la troisième sous étape SE3 il est déterminé une correspondance entre intensités de courant et concentrations en acide nucléique.

Cette correspondance permet d'associer, au moins sur une certaine plage d'intensités et une certaine plage de concentrations, à une intensité de courant une concentration en acide nucléique et inversement à une concentration en acide nucléique une intensité de courant.

Cette correspondance peut être déterminée par exemple en effectuant une régression linéaire des intensités de référence en fonction de la concentration en acide nucléique.

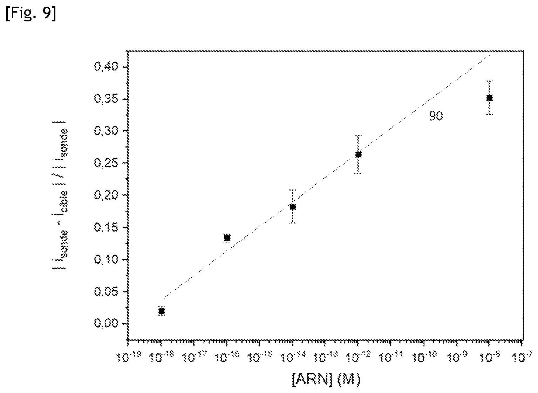

La figure 9 illustre une autre correspondance possible dans laquelle une intensité relative est déterminée avant d'effectuer la régression linéaire.

Cette intensité relative est construite à partir d'une intensité de sonde définie comme l'intensité de référence pour la solution de référence ne contenant pas d'acides nucléiques.

L'intensité relative est définie comme la valeur absolue du rapport de la différence entre une intensité de référence et l'intensité de sonde sur l'intensité de sonde.

Les valeurs expérimentales issues de l'expérience en rapport avec la figure 8 sont utilisées pour construire les points de la figure 9.

Une régression linéaire de ces points est ensuite effectuée pour obtenir une courbe d'ajustement 90.

La courbe d'ajustement qui modélise l'intensité relative en fonction de la concentration en acides nucléiques constitue une correspondance comme recherchée.

L'étape de calibration est achevée lorsque la correspondance entre intensités de courant et concentrations en acide nucléique est obtenue.

Le procédé de détection d'acide nucléique peut être affiné pour fournir une estimation de la concentration en acide nucléique grâce à la correspondance.

L'intensité mesurée pour la solution à analyser dont la concentration en acides nucléiques est inconnue constitue la valeur d'entrée dans la correspondance. La correspondance permet d'associer à cette valeur d'entrée une valeur de sortie qui est une concentration en acide nucléique. Cette concentration constitue alors une estimation de la concentration en acides nucléiques dans la solution à analyser.

Le procédé de détection tel que décrit ci-avant peut notamment être mis en œuvre lorsque l'acide nucléique cible est un fragment d'acide nucléique d'un agent pathogène, par exemple un fragment d'acide nucléique ARN codant pour un coronavirus. Un tel procédé peut être utile dans le domaine du diagnostic rapide pour la biologie d'urgence. Le procédé décrit permet de réaliser des détections de ces fragments à l'état de trace, ce qui est impossible en utilisant les technologies électrochimiques fondées sur l'utilisation d'une électrode en or.

De plus, il est possible avec le procédé décrit d'obtenir des mesures quantitatives et absolues sans faire appel à une technique PCR - réaction en chaine par polymérase - qui suppose une transcription inverse -RT - d'ARN en ADN, et une amplification des brins ADN permettant de les amener à un seuil détectable.

## Revendications

1. Procédé de fixation d'acide nucléique sur une électrode (5, 20) de platine comprenant les étapes suivantes :
- une étape (S1) de fixation d'une molécule d'éthylènediamine (22) sur l'électrode (5, 20), l'étape (S1) comprenant une électro-oxydation d'une amine primaire de la molécule d'éthylènediamine (22) par voltammétrie cyclique,
- une étape (S2) de fixation d'une molécule sulfo SMMC (26) de 4-(N-Maleimidomethyl)cyclohexane-1-carboxylic acid 3- sulfo-N-hydroxysuccinimide ester sur la molécule d'éthylènediamine (22),
- une étape (S3) de fixation d'un acide nucléique (32) sur la molécule sulfo SMMC (26), l'acide nucléique (32) étant préalablement modifié pour comprendre une fonction thiol, l'électrode (5, 20) étant en contact avec une solution aqueuse, c'est-à-dire avec une solution dont le solvant est l'eau, durant les étapes (S1), (S2) et (S3).

2. Procédé selon la revendication 1 dans lequel la solution aqueuse est une solution physiologique.

3. Procédé selon l'une des revendications 1 à 2 dans lequel l'électrode (5, 20) est une micro-électrode placée au moins en partie dans un canal micro-fluidique.

4. Procédé selon l'une des revendications 1 à 3 comprenant après l'étape (S3) une étape (S4) de stabilisation pendant laquelle l'électrode (5, 20) est placée en solution physiologique durant une durée comprise entre 20 minutes et 40 minutes, la solution présentant une concentration en NaCl comprise entre 0,4 molaire et 0,6 molaire.

5. Electrode (20) de platine pour la détection d'un acide nucléique cible (36) comprenant une molécule d'éthylènediamine (22) fixée sur l'électrode (5, 20), une molécule sulfo SMMC (26) de 4-(N-Maleimidomethyl)cyclohexane-1-carboxylic acid 3- sulfo-N-hydroxysuccinimide ester étant fixée sur la molécule d'éthylènediamine (22) par électro-oxydation d'un amine primaire de la molécule d'éthylènediamine (22) par voltammétrie cyclique, un acide nucléique sonde (32) étant fixé sur la molécule sulfo SMMC (26), l'acide nucléique sonde (32) comprenant une fonction thiol, l'acide nucléique sonde (32) étant complémentaire de l'acide nucléique cible (36).

6. Dispositif (100) de détection d'un acide nucléique cible comprenant une électrode de platine (5) selon la revendication 5, une contre-électrode (6) et un système de mesures électriques relié électriquement à l'électrode (5) de platine et à la contre-électrode (6).

7. Dispositif selon la revendication 6, le dispositif comprenant en outre un canal micro-fluidique (4), l'électrode de platine (5) étant une micro-électrode, l'électrode de platine (5) et la contre-électrode (6) étant placées au moins en partie dans le canal micro-fluidique (4).

8. Système (200) de détection d'acides nucléiques cibles comprenant une pluralité de dispositifs (100) selon la revendication 6 ou 7, le système (200) comprenant une entrée configurée pour recevoir une solution à analyser, l'entrée étant reliée à chaque électrode de platine de chaque dispositif.

9. Système (200) de détection selon la revendication 8, deux dispositifs de la pluralité de dispositifs étant configurés pour détecter deux acides nucléiques cibles différents.

10. Procédé de détection d'un acide nucléique cible dans une solution à analyser, le procédé comprenant les étapes suivantes :
- une étape (E1) de fourniture d'une électrode de travail, l'électrode de travail étant une électrode en platine selon la revendication 5,
- une étape (E2) de fourniture d'une contre-électrode,
- une étape (E3) de mise sous tension électrique de l'électrode de travail et de la contre-électrode de sorte à maintenir une tension de travail entre elles,
- une étape (E4) de mise en contact de l'électrode de travail et de la contre-électrode avec la solution à analyser,
- une étape (E5) de détermination d'une intensité mesurée d'un courant électrique entre l'électrode de travail et la contre-électrode,
- une étape (E6) de détermination d'une présence d'un acide nucléique cible dans la solution à analyser à partir de l'intensité mesurée.

11. Procédé selon la revendication 10 comprenant en outre une étape de calibration comprenant les sous-étapes suivantes :
- une première sous-étape (SE1) de fourniture de deux solutions de référence présentant des concentrations différentes en acide nucléique cible,
- une deuxième sous-étape (SE2) de mesure pour chaque solution de référence d'une intensité de référence entre l'électrode de travail et la contre-électrode, les électrodes étant mises en contact avec la solution de référence, les électrodes étant mises sous tension électrique de sorte à maintenir la tension de travail entre elles,
- une troisième sous étape (SE3) de détermination d'une correspondance d'intensités de courant entre l'électrode de travail et la contre-électrode en fonction et de concentrations en acide nucléique cible dans une solution à analyser,
le procédé comprenant une étape de détermination d'une concentration en acide nucléique cible dans la solution à analyser à partir de l'intensité mesurée utilisant la correspondance.

12. Procédé selon l'une des revendications 10 ou 11, dans lequel l'acide nucléique cible est un fragment d'acide nucléique d'un agent pathogène.

## Patentansprüche

1. Verfahren zum Fixieren von Nukleinsäure an einer Platinelektrode (5, 20), umfassend die folgenden Schritte:
- einen Schritt (S1) zum Fixieren eines Ethylendiaminmoleküls (22) an der Elektrode (5, 20), wobei der Schritt (S1) eine Elektrooxidation eines primären Amins des Ethylendiaminmoleküls (22) durch zyklische Voltammetrie umfasst,
- einen Schritt (S2) zum Fixieren eines Sulfo-SMMC-Moleküls (26) von 4-(N-Maleimidomethyl)cyclohexan-1-carboxylsäure-3-sulfo-N-hydroxysuccinimidester an das Ethylendiaminmolekül (22),
- einen Schritt (S3) zum Fixieren einer Nukleinsäure (32) an das Sulfo-SMMC-Molekül (26), wobei die Nukleinsäure (32) zuvor so modifiziert wurde, dass sie eine Thiolfunktion umfasst, wobei die Elektrode (5, 20) während der Schritte (S1), (S2) und (S3) mit einer wässrigen Lösung, d. h. mit einer Lösung, deren Lösungsmittel Wasser ist, in Kontakt steht.

2. Verfahren nach Anspruch 1, wobei die wässrige Lösung eine physiologische Lösung ist.

3. Verfahren nach einem der Ansprüche 1 bis 2, wobei die Elektrode (5, 20) eine mindestens teilweise in einem mikrofluidischen Kanal platzierte Mikroelektrode ist.

4. Verfahren nach einem der Ansprüche 1 bis 3, das nach Schritt (S3) einen Schritt (S4) zum Stabilisieren umfasst, bei dem die Elektrode (5, 20) für eine Dauer zwischen 20 Minuten und 40 Minuten in Kochsalzlösung gelegt wird, wobei die Lösung eine NaCl-Konzentration zwischen 0,4 Mol und 0,6 Mol aufweist.

5. Platinelektrode (20) zum Erkennen einer Ziel-Nukleinsäure (36), die ein an der Elektrode (5, 20) fixiertes Ethylendiaminmolekül (22) umfasst, wobei ein Sulfo-SMMC-Molekül (26) von 4-(N-Maleimidomethyl)cyclohexan-1-carboxylsäure-3-sulfo-N-hydroxysuccinimidester an dem Ethylendiaminmolekül (22) durch Elektrooxidation eines primären Amins des Ethylendiaminmoleküls (22) durch zyklische Voltammetrie fixiert ist, wobei eine Sonden-Nukleinsäure (32) an dem Sulfo-SMMC-Molekül (26) fixiert ist, wobei die Sonden-Nukleinsäure (32) eine Thiolfunktion umfasst, wobei die Sonden-Nukleinsäure (32) komplementär zu der Ziel-Nukleinsäure (36) ist.

6. Vorrichtung (100) zum Erkennen einer Ziel-Nukleinsäure, umfassend eine Platinelektrode (5) nach Anspruch 5, eine Gegenelektrode (6) und ein elektrisches Messsystem, das elektrisch mit der Platinelektrode (5) und der Gegenelektrode (6) verbunden ist.

7. Vorrichtung nach Anspruch 6, wobei die Vorrichtung ferner einen mikrofluidischen Kanal (4) umfasst, wobei die Platinelektrode (5) eine Mikroelektrode ist, wobei die Platinelektrode (5) und die Gegenelektrode (6) mindestens teilweise in dem mikrofluidischen Kanal (4) platziert sind.

8. System (200) zum Erkennen von Ziel-Nukleinsäuren, umfassend eine Vielzahl von Vorrichtungen (100) nach Anspruch 6 oder 7, wobei das System (200) einen Eingang umfasst, der so konfiguriert ist, dass er eine zu analysierende Lösung empfängt, wobei der Eingang mit jeder Platinelektrode jeder Vorrichtung verbunden ist.

9. System (200) zum Erkennen nach Anspruch 8, wobei zwei Vorrichtungen der Vielzahl von Vorrichtungen so konfiguriert sind, dass sie zwei unterschiedliche Nukleinsäureziele erkennen.

10. Verfahren zum Erkennen einer Ziel-Nukleinsäure in einer zu analysierenden Lösung, wobei das Verfahren die folgenden Schritte umfasst:
- einen Schritt (E1) zum Bereitstellen einer Arbeitselektrode, wobei die Arbeitselektrode eine Platinelektrode nach Anspruch 5 ist,
- einen Schritt (E2) zum Bereitstellen einer Gegenelektrode,
- einen Schritt (E3) zum elektrischen Einschalten der Arbeitselektrode und der Gegenelektrode, so dass eine Arbeitsspannung zwischen ihnen aufrechterhalten wird,
- einen Schritt (E4) zum Inkontaktbringen der Arbeitselektrode und der Gegenelektrode mit der zu analysierenden Lösung,
- einen Schritt (E5) zum Bestimmen einer gemessenen Stromstärke eines elektrischen Stroms zwischen der Arbeitselektrode und der Gegenelektrode,
- einen Schritt (E6) zum Bestimmen des Vorhandenseins einer Ziel-Nukleinsäure in der zu analysierenden Lösung anhand der gemessenen Stromsstärke.

11. Verfahren nach Anspruch 10, ferner umfassend einen Schritt zum Kalibrieren, der die folgenden Unterschritte umfasst:
- einen ersten Unterschritt (SE1) zum Bereitstellen von zwei Referenzlösungen mit unterschiedlichen Konzentrationen an Ziel-Nukleinsäure,
- einen zweiten Unterschritt (SE2) zum Messen für jede Referenzlösung einer Referenzstromstärke zwischen der Arbeitselektrode und der Gegenelektrode, wobei die Elektroden mit der Referenzlösung in Kontakt gebracht und die Elektroden unter elektrischer Spannung gesetzt werden, um die Arbeitsspannung zwischen ihnen aufrechtzuerhalten,
- einen dritten Unterschritt (SE3) zum Bestimmen einer Übereinstimmung von Stromstärken zwischen der Arbeitselektrode und der Gegenelektrode in Abhängigkeit von und Konzentrationen der Ziel-Nukleinsäure in einer zu analysierenden Lösung,
wobei das Verfahren einen Schritt zum Bestimmen einer Ziel-Nukleinsäurekonzentration in der zu analysierenden Lösung anhand der gemessenen Stromstärke unter Verwendung der Übereinstimmung umfasst.

12. Verfahren nach einem der Ansprüche 10 oder 11, wobei die Zielnukleinsäure ein Nukleinsäurefragment eines Krankheitserregers ist.

## Claims

1. Method for attaching a nucleic acid to a platinum electrode (5, 20) comprising the following steps:
- a step (S1) of attaching an ethylenediamine molecule (22) to the electrode (5, 20), the step (S1) comprising electro-oxidation of a primary amine of the ethylenediamine molecule (22) by cyclic voltammetry,
- a step (S2) of attaching a sulfo SMMC molecule (26) of 4-(N-Maleimidomethyl)cyclohexane-1-carboxylic acid 3-sulfo-N-hydroxysuccinimide ester to the ethylenediamine molecule (22),
- a step (S3) of attaching a nucleic acid (32) to the sulfo SMMC molecule (26), the nucleic acid (32) being previously modified to comprise a thiol function, the electrode (5, 20) being in contact with an aqueous solution, that is to say with a solution of which the solvent is water, during steps (S1), (S2) and (S3).

2. Method according to claim 1, wherein the aqueous solution is a physiological solution.

3. Method according to one of claims 1 to 2, wherein the electrode (5, 20) is a micro-electrode placed at least partially in a micro-fluidic channel.

4. Method according to one of claims 1 to 3 comprising after step (S3) a stabilisation step (S4) during which the electrode (5, 20) is placed in a physiological solution for a duration of between 20 minutes and 40 minutes, the solution having a NaCl concentration between 0.4 molar and 0.6 molar.

5. Platinum electrode (20) for detecting a target nucleic acid (36) comprising an ethylenediamine molecule (22) attached to the electrode (5, 20), a sulfo SMMC molecule (26) of 4-(N-maleimidomethyl)cyclohexane-1-carboxylic acid 3-sulfo-N-hydroxysuccinimide ester being attached to the ethylenediamine molecule (22) by electro-oxidation of a primary amine of the ethylenediamine molecule (22) by cyclic voltammetry, a probe nucleic acid (32) being attached to the sulfo SMMC molecule (26), the probe nucleic acid (32) comprising a thiol function, the probe nucleic acid (32) being complementary to the target nucleic acid (36).

6. Device (100) for detecting a target nucleic acid comprising a platinum electrode (5) according to claim 5, a counter electrode (6) and an electrical measurement system electrically connected to the platinum electrode (5) and to the counter electrode (6).

7. Device according to claim 6, the device further comprising a micro-fluidic channel (4), the platinum electrode (5) being a micro-electrode, the platinum electrode (5) and the counter electrode (6) being placed at least partially in the micro-fluidic channel (4).

8. System (200) for detecting target nucleic acids comprising a plurality of devices (100) according to claim 6 or 7, the system (200) comprising an inlet configured to receive a solution to be analysed, the inlet being connected to each platinum electrode of each device.

9. Detection system (200) according to claim 8, two devices of the plurality of devices being configured to detect two different target nucleic acids.

10. Method for detecting a target nucleic acid in a solution to be analysed, the method comprising the following steps:
- a step (E1) of providing a working electrode, the working electrode being a platinum electrode according to claim 5,
- a step (E2) of providing a counter electrode,
- a step (E3) of electrically energising the working electrode and the counter electrode so as to maintain a working voltage between them,
- a step (E4) of placing the working electrode and the counter electrode in contact with the solution to be analysed,
- a step (E5) of determining a measured intensity of an electrical current between the working electrode and the counter electrode,
- a step (E6) of determining a presence of a target nucleic acid in the solution to be analysed based on the measured intensity.

11. Method according to claim 10 further comprising a calibration step comprising the following sub-steps:
- a first sub-step (SE1) of providing two reference solutions having different target nucleic acid concentrations,
- a second sub-step (SE2) of measuring for each reference solution a reference intensity between the working electrode and the counter electrode, the electrodes being placed in contact with the reference solution, the electrodes being electrically energised so as to maintain the working voltage between them,
- a third sub-step (SE3) of determining a correspondence of current intensities between the working electrode and the counter electrode in operation and target nucleic acid concentrations in a solution to be analysed,
the method comprising a step of determining a target nucleic acid concentration in the solution to be analysed based on the measured intensity using the correspondence.

12. Method according to any one of claims 10 or 11, wherein the target nucleic acid is a nucleic acid fragment of a pathogenic agent.
